# EUROPÄISCHE PATENTSCHRIFT

(11) **EP 1 750 700 B1**
(45) Veröffentlichungstag und Bekanntmachung des Hinweises auf die Patenterteilung: **02.01.2008**
(21) Anmeldenummer: 05748118.6
(22) Anmeldetag: 04.05.2005
(51) Int. Cl.: A61K 31/43, A61K 31/5415, A61P 29/00, A61P 31/00

(54) **FLÜSSIGE ZUBEREITUNG FÜR DIE VETERINÄRMEDIZIN; VERFAHREN ZU DEREN HERSTELLUNG UND DEREN VERWENDUNG**
LIQUID PREPARATION FOR VETERINARY MEDICINE, METHOD FOR THE PRODUCTION THEREOF, AND USE OF THE SAME
PREPARATION LIQUIDE DESTINEE A LA MEDECINE VETERINAIRE, PROCEDE DE FABRICATION ET UTILISATION DE LADITE PREPARATION

(30) Priorität: 19.05.2004 DE 102004025324
(43) Veröffentlichungstag der Anmeldung: 14.02.2007
(73) Patentinhaber: Boehringer Ingelheim Vetmedica GmbH, 55216 Ingelheim am Rhein (DE)
(72) Erfinder: FOLGER, Martin, 55218 Ingelheim (DE); HASSEL, Bernhard, 55437 Ockenheim (DE); HENKE, Stefan, 57548 Kirchen (DE); JANOTT, Dietrich, W., 55218 Ingelheim (DE); RIECHEL, Peter, 55218 Ingelheim (DE)
(74) Vertreter: Hammann, Heinz
(86) Internationale Anmeldenummer: PCT/EP2005/004824
(87) Internationale Veröffentlichungsnummer: WO 2005/115386

(56) Entgegenhaltungen:
- WO-A-01/97813
- GB-A- 2 087 236

## Beschreibung

Die Erfindung betrifft eine flüssige Zubereitung für die Veterinärmedizin, ein Verfahren zu deren Herstellung und deren Verwendung.

### Hintergrund der Erfindung

Aufgrund der Züchtungen in den letzten Jahrzehnten, wo versucht wurde, bei Kühen eine leichtere Melkbarkeit zu erhalten, zeigt sich nunmehr die Kehrseite dieser Züchtungen in zunehmenden Problemen mit Euterentzündungen (Mastitis). Die Mastitis des Rinds ist in Gebieten mit intensiver Milchproduktion neben Fruchtbarkeitsstörungen weltweit der häufigste Grund für eine vorzeitige Schlachtung von Milchkühen. Daher steigt die Bedeutung von Arzneimitteln, die in diesem Bereich eingesetzt werden.

So ist Meloxicam ein nicht-steroidaler, entzündungshemmender Wirkstoff aus der Oxicam-Klasse und wird auch als meloxicamhaltige Lösung (Handelsname: Metacam®) vertrieben. Es handelt sich um einen bekannten Cyclooxygenasen (COX-I/COX-II) Inhibitor, der folgende chemische Struktur aufweist:

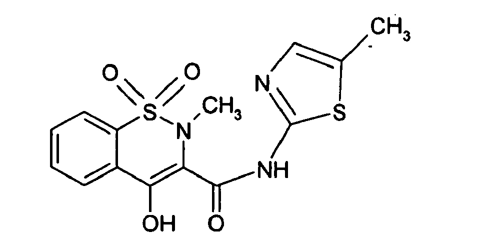

Dieses nichtsteroidale Antiphlogistikum ist unter anderem für die Behandlung von Mastitiden der Milchkuh zugelassen, d.h. es wirkt gegen Entzündungen im Euter. Dieses Arzneimittel dient auch bei akuten Fällen und wird insbesondere zur Vermeidung von Langzeit-Entzündungsschäden im Euter eingesetzt. Meloxicam kommt im tiermedizinischen Bereich vor allem als Injektionslösung zum Einsatz, wobei der Wirkstoff nach subkutaner Verabreichung vollständig bioverfügbar ist. Maximale Plasmakonzentrationen werden nach einigen Stunden erreicht. Eine einmalige Injektion ist in der Regel bereits ausreichend.

Da sämtliche Hauptmetabolite des Meloxicam pharmakologisch inaktiv sind, bestimmt sich die Wartezeit nach erfolgter Injektion ausschließlich über die Konzentration von aktivem Wirkstoff im entsprechenden Gewebe und beträgt bei Metacam® für essbare Gewebe bei Rindern und Kühen 15 Tage und für Milch 5 Tage. Bei Schweinen ist eine Wartezeit von 5 Tagen einzuhalten.

Weitere Indikationen dieses Wirkstoffs bei Rind und Schwein sind Atemwegserkrankungen bei Rindern und Kühen, Durchfallerkrankungen bei Kälbern, akute Mastitis der Milchkuh, MMA (Mastitis-Metritis-Agalactica-Syndrom) des Schweins und nicht infektiöse Lahmheiten beim Schwein.

Als Ursache der akuten Mastitis wurden Streptokokken, Staphylokokken und E. coli diagnostiziert. Daher erfolgt die Behandlung mit Meloxicam häufig in Kombination mit einem Antibiotikum, wodurch sich die klinischen Symptome noch signifikanter verbessern und die lokale Entzündung des Eutergewebes schnell und nachhaltig gehemmt wird. Grundsätzlich sind für die antibakterielle Mastitistherapie beispielsweise β-Lactam-Antibiotika, Aminoglykoside, Lincosamide, Makrolide, Cephalosporine, Tetracycline, Penicilline, Polypeptide, Trimethoprim-Sulfonamid-Kombinationen und Fluochinolone geeignet.

Es ist daher von Nutzen, gleichzeitig mit Meloxicam eine geeignete Antibiotika-Therapie durchzuführen. Aus diesem Grund gibt es im Stand der Technik eine Vielzahl von Vorschlägen in den unterschiedlichsten medizinischen Bereichen, Meloxicam zusammen mit Antibiotika zu verabreichen.

So beschreibt die WO O1/015687 eine Zusammensetzung eines COX-II selektiven Inhibitors, wie Meloxicam, optional in Kombination mit weiteren aktiven Mitteln, wobei auch Antibiotika genannt sind, zur Behandlung oder Vorbeugung für Prostatitis oder das chronische Schmerzsyndrom im kleinen Becken.

Gemäß der WO 93/01814 wird eine einträufelbare ophthalmologische Zusammensetzung beschrieben, die ein entzündungshemmendes Mittel aus der Gruppe der Oxicame zusammen mit einem gelbildenden Acrylsäurepolymer und einem basischen Mittel enthält, wobei ein antibakterielles Mittel, wie ein Antibiotikum, zusätzlich vorhanden sein kann.

Die WO 98/06385 und die EP 0 918 513 betreffen jeweils leicht schluckbare pharmazeutische Zusammensetzungen zur oralen Verabreichung, enthaltend mindestens einen Wirkstoff, die mit entsprechenden Überzügen versehen sind. Unter einer großen Vielzahl von möglichen Wirkstoffen werden auch Meloxicam und Antibiotika genannt.

WO 01/97813 beschreibt stabile flüssige Darreichungsfomen mit Meloxicam und dessen mögliche Kombination mit Antibiotika, sowie eine tiermedizinische Anwendung.
GB-A-2087236 beschreibt Darreichungsformen mit Penethamathhydrojodid für die Veterinärmedizin.

Die Entwicklung von speziellen Kombinationspräparaten ist daher stets von großem Interesse. Aufgrund der Bedeutung der Gesundheit von Nutztieren für den menschlichen Konsum besteht ständig ein Bedürfnis danach, Möglichkeiten aufzufinden, um die Krankheiten dieser Tiere noch effektiver zu behandeln oder diesen vorzubeugen und hierfür geeignete Arzneimittel bereitzustellen.

Der vorliegenden Erfindung liegt somit die Aufgabe zugrunde, eine Zubereitung zur Verfügung zu stellen, mit der eine Verabreichung von Meloxicam zusammen mit einem Antibiotikum in einfacher Weise erfolgen kann. Es soll eine Darreichungsform entwickelt werden, mit der eine Kombination Meloxicam/Antibiotikum bereitgestellt wird, wobei beide Wirkstoffe in ihrem Wirkungsspektum nicht nachteilig beeinflusst werden sollen. Ferner soll ein Verfahren zur Verfügung gestellt werden, mit dem diese Darreichungsform in einfacher Weise hergestellt werden kann. Zudem soll die zu entwickelnde Darreichungsform eine einfache Verabreichung an Tiere bereitstellen.

### Detaillierte Beschreibung der Erfindung

Die vorstehend genannte Aufgabe wird durch die Merkmale des Anspruchs 1 gelöst. Hiernach wird eine flüssige Zubereitung für die Veterinärmedizin bereitgestellt, enthaltend
- als ersten Wirkstoff Meloxicam,
- als zweiten Wirkstoff Penethamathydrojodid und
- mindestens ein Lösungsmittel.

Demzufolge wird ein erster Wirkstoff, d.h. Meloxicam, in Kombination mit einem speziellen Antibiotikum, d.h. Penethamathydrojodid, als zweitem Wirkstoff, in Form einer flüssigen Zubereitung zur Verfügung gestellt. Der zweite Wirkstoff, das Penethamathydrojodid, auch bekannt unter "Benzylpenicillathydrojodid" und "Penethacillinhydrojodid", ist ein Wirkstoff, welcher in der Behandlung von Mastidien beim Rind, ausgelöst durch Streptokokken und nicht β-Laktamase-bildende Staphylokokken sowie andere Penicillin-empfindliche Erreger, eingesetzt wird. Dieser Wirkstoff wird als intramuskuläres Mastitistherapeutikum unter dem Handelsnamen Ingel-Mamyzin® vertrieben, wobei der Wirkstoff Penethamathydrojodid als Trockensubstanz und das Lösungsmittel in Form von Wasser, konserviert mit Methyl-4-hydroxybenzoat, getrennt voneinander geliefert und beide Komponenten erst unmittelbar vor Gebrauch vermischt werden. Die Haltbarkeitsdauer der Injektionslösung von Ingel-Mamyzin® nach dem Zusammenmischen beider Komponenten, d.h der Trockensubstanz und dem Lösungsmittel, beträgt bei einer Aufbewahrungstemperatur zwischen 2 und 8°C 7 Tage und bei einer Aufbewahrungstemperatur im Bereich zwischen 15 und 25°C 2 Tage.

Es ist daher in überraschender Weise mit der vorliegenden Erfindung gelungen mit einem einfachen Austausch des Lösungsmittels im Ingel-Mamyzin® durch eine Meloxicam-Lösung eine besonders wirksame flüssige Zubereitung zu erhalten. Die Behandlung von Säugetieren mit einer derartigen Mischung war bisher nicht möglich, da eine Meloxicam-Lösung einen pH-Wert um 9 aufweist und daher angenommen wurde, dass keine stabilen Zusammensetzungen erhalten werden könnten. In Versuchen, die bei den Beispielen im Einzelnen erläutert werden, konnte jedoch in unerwarteter Weise belegt werden, dass eine ausreichende Stabilität erhalten wird.

Die flüssige Zubereitung der Erfindung ermöglicht den gleichzeitigen Einsatz von beiden Wirkstoffen, die beide ungehindert ihre Wirkungen entfalten.

Die flüssige Zubereitung wird in Form einer Suspension erhalten. Es handelt sich um ein disperses System. Unter "dispersem System" versteht man ganz allgemein ein aus zwei oder mehreren Phasen bestehendes System, bei welchem eine Formart (disperse Phase) in der anderen Formart (Dispersionsmittel) fein verteilt ist. Unter einer "Suspension" wird erfindungsgemäß ein Gemisch von Feststoffteilchen in einer Flüssigkeit verstanden.

Die Suspension ist bevorzugt homogen, d.h. unmittelbar nach dem Schütteln stellt sich die Suspension dem Betrachter mit nahezu einheitlichem Erscheinungsbild dar. "Flüssige Zubereitung" schließt jedoch nicht aus, dass sich auch ein Niederschlag aus Feststoffteilchen bilden kann, der bei längerem Stehenlassen anwächst und bei erneutem Schütteln zum Teil oder gänzlich wieder verschwindet. Der Begriff "flüssige Zubereitung", wie er in der vorliegenden Erfindung verwendet wird, bedeutet nur, dass die Hauptmenge der Zubereitung in flüssiger Form vorliegt.

Es hat sich als vorteilhaft erwiesen, wenn das Meloxicam in der flüssigen Zubereitung im Lösungsmittel gelöst vorliegt. "Gelöst" soll in diesem Zusammenhang nicht nur eine echte Lösung bedeuten, sondern umfasst auch ein disperses System, insbesondere auch ein kolloiddisperses System.

Zweckmäßigerweise wird das Lösungsmittel in der flüssigen Zubereitung ausgewählt aus der Gruppe, bestehend aus Alkohol, Wasser, und/oder Alkohol/Wasser, wobei Ethanol besonders bevorzugt eingesetzt wird. Ganz besonders bevorzugt werden Ethanol/Wasser-Gemische mit einem Verhältnis Ethanol: Wasser (w/w) von 1:7 bis 1:5,7, bevorzugt 1:6 bis 1:5,7, besonders bevorzugt 1:5,7, verwendet.

Nach einer bevorzugten Ausführungsform weist die Zubereitung 0,2 bis 0,5, insbesondere 0,34 bis 0,4 g Penethamathydrojodid pro ml Lösung auf. Ganz besonders bevorzugt wird das Penethamathydrojodid als Trockensubstanz eingesetzt. "Trockensubstanz" bedeutet, dass das Penethamathydrojodid in getrockneter Form eingesetzt wird, wie es als eine Komponente des Ingel-Mamyzins® bereits erhältlich ist.

Ebenfalls bevorzugt ist die Verwendung einer Lösung, welche einem Dosierungsbereich von 0,2 bis 0,6 mg, vorzugsweise 0,5 mg, Meloxicam /kg Körpergewicht und 8 bis 12 mg, vorzugsweise 10 mg, Penethamathydroiodid /kg Körpergewicht entspricht.

Es ist weiterhin bevorzugt, wenn Meloxicam in der flüssigen Zubereitung der Erfindung (Endformulierung) in einer Menge von 0,2 g bis 0,4 g pro ml Endformulierung, insbesondere 0,25 bis 0,32 g pro ml Endformulierung vorhanden ist.

Selbstverständlich können auch Hilfsstoffe enthalten sein, die im Rahmen der Erfindung nicht besonders beschränkt sind. Lediglich beispielhaft seien die Hilfsstoffe einer handelsüblichen Meloxicam-Injektionslösung angegeben mit: Meglumin, Macrogol 30D, Poloxamer 188, ED-TA-Na und Glycin. Selbstverständlich können auch andere dem Fachmann bekannte Hilfsstoffe verwendet werden.

Es ist besonders vorteilhaft, wenn die flüssige Zubereitung in Form einer intramuskulären Injektionslösung, gegebenenfalls mit entsprechenden Hilfsstoffen, hergestellt wird. Dies erlaubt eine genaue Dosierung, einfache Verabreichung und schnelle Wirksamkeit.

Eine ganz besonders bevorzugte flüssige Zubereitung der Erfindung enthält
- 0,25 bis 0,31 g Meloxicam pro ml Endformulierung,
- 0,40 bis 0,30 g Penethamathydrojodid pro ml Endformulierung,
- 1,88 bis 0,34 ml Ethanol und
- 7,74 bis 0,67 ml Wasser.

Gegenstand der Erfindung ist ebenfalls ein Verfahren zur Herstellung der oben beschriebenen flüssigen Zubereitung, umfassend die Schritte:
(a) Lösen von Meloxicam in einem Lösungsmittel;
(b) Zugeben von Penethamathydrojodid zu der in Schritt (a) erhaltenen Lösung und
(c) Schütteln der in Schritt (b) erhaltenen Mischung unter Erhalt der flüssigen Zubereitung in Form einer Suspension.

Das erfindungsgemäße Verfahren ermöglicht somit in einfacher Art und Weise ein Kombinationspräparat zur Verfügung zu stellen, wobei ein Zusammengeben beider Komponenten und anschließendes Schütteln der Mischung die zum Gebrauch fertige Zubereitung liefert.

Zunächst wird Meloxicam in einem Lösungsmittel gelöst, wobei "gelöst" im Sinne der Erfindung, wie oben definiert, auch "dispergiert" umfasst. Es ist besonders bevorzugt, wenn die in Schritt (a) erhaltene Lösung von Meloxicam in einem Lösungsmittel, wie Ethanol und gegebenenfalls Wasser, 1 bis 10%ig, insbesondere 1 bis 5%ig hergestellt wird. Ganz besonders bevorzugt wird von einer 2%igen Meloxicam-Lösung ausgegangen. Selbstverständlich kann auch eine handelsübliche Meloxicam-Injektionslösung eingesetzt werden.

Im Anschluß wird im erfindungsgemäßen Schritt (b) das Penethamathydrojodid in der erforderlichen Menge zugegeben, wie bereits erläutert wurde, wobei dieses bevorzugt als Trockensubstanz eingesetzt wird.

Selbstverständlich können in Schritt (a) und/oder Schritt (b) die bereits erläuterten Hilfsstoffe in den angegebenen Mengenbereichen zugesetzt werden.

Vorteilhafterweise wird die flüssige Zubereitung der Erfindung unmittelbar vor Gebrauch hergestellt. Die Haltbarkeitsdauer nach dem Zusammenmischen beträgt bei einer Aufbewahrungstemperatur zwischen 2 und 8 °C 7 Tage.

Die Erfindung betrifft ebenfalls die Verwendung der flüssigen Zubereitung zur Herstellung eines Arzneimittels zur gleichzeitigen Behandlung oder Vorbeugung von Krankheiten bei Tieren, ausgewählt aus den bekannten Indikationen für Meloxicam und/oder Penethamathydrojodid, vorzugsweise ausgewählt aus den Indikationen .Atemwegserkrankungen bei Rindern und Kühen, Durchfallerkrankungen bei Kälbern, Mastitis, akuter Mastitis der Milchkuh, Mastitis-Metritis-Agalactica-Syndrom des Schweins, nicht infektiösen Lahmheiten beim Schwein, Prostatitis und dem chronischen Schmerzsyndrom im kleinen Becken.

Eine Indikation ist die Behandlung von Mastitis. Jedoch können gleichzeitig auch zwei Indikationen behandelt werden, wie Mastitis und eine Atemwegsinfektion.

Die zu behandelnden Tiere sind insbesondere Säugetiere, wie Rinder oder Schweine. Die flüssige Zubereitung findet dann bevorzugt als intramuskuläre Injektionslösung Verwendung.

Die mit der vorliegenden Erfindung verbundenen Vorteile sind vielschichtig:
Erfindungsgemäß wird erstmals eine Mischung von Meloxicam und Penethamathydrojodid zur Verfügung gestellt. Die Behandlung von Säugetieren mit dieser Mischung war bisher nicht möglich, da keine stabile Darreichungsform hergestellt werden konnte.

Die erfindungsgemäße Zubereitung ist ausreichend stabil, was überraschend ist, da zum Beispiel eine 2%ige Meloxicam-Lösung einen pH-Wert von 8,9 hat und trotzdem eine sehr gute Gebrauchsstabilität vorliegt.

Dieses Kombinationspräparat in Form der flüssigen Zubereitung der Erfindung ermöglicht den gleichzeitigen Einsatz von beiden Wirkstoffen, wobei es möglich wird, zwei Indikationen, wie Mastitis und Atemwegsinfektionen, gleichzeitig zu therapieren. Durch die Anwendung beider Wirkstoffe kann die Menge an Antibiotikum verringert werden, verglichen mit der Menge an Antibiotikum, die separat verabreicht werden müßte, was auf einen synergistischen Effekt hindeutet. Ein Vorteil bei Verringerung der notwendigen Menge an Antibiotikum ist beispielweise eine deutlich geringere Belastung mit Antibiotikum der behandelten Nutztiere, wodurch die üblichen Risiken, wie Entstehung von Allergien und Resistenzausbildung, deutlich abgeschwächt werden.

Ferner kann die Zubereitung einfach mit den bestehenden Handelsformen hergestellt werden. Es kann auf eine Komponente des Ingel-Mamyzins®, d.h. das Penethamathydrojodid in Form der Trockensubstanz, zurückgegriffen werden, und es kann eine handelsübliche Meloxicam-Lösung herangezogen werden.

Darüber hinaus wird ein einfaches Herstellungsverfahren zur Verfügung gestellt, indem durch Zusammengeben und Schütteln die flüssige Zubereitung der Erfindung sofort bereitsteht. Die Verabreichung als Injektionslösung bietet durch die exakte Dosierung, unmittelbare Bioverfügbarkeit der Wirkstoffe und einfache Verabreichung, insbesondere bei der Behandlung von Tieren, Vorteile.

Die nachfolgenden Beispiele dienen der Illustration erfindungsgemäßer Formulierungen. Sie sind lediglich als mögliche, exemplarisch dargestellte Vorgehensweisen zu verstehen, ohne die Erfindung auf deren Inhalt zu beschränken.

### Beispiele

### Beispiel 1

Zur Untersuchung der Stabilität wurden mikroskopische Aufnahmen von einer Ausführungsform der erfindungsgemäßen flüssigen Zubereitung und einer Formulierung aus dem Stand der Technik aufgenommen. Diese sind in den Figuren 1 bis 5 wiedergegeben. Im Einzelnen zeigt:
- Figur 1: eine mikroskopische Aufnahme einer Ingel-Mamyzin®-Suspension;
- Figur 2: eine mikroskopische Aufnahme einer Ausführungsform der erfindungsgemäßen Zubereitung direkt nach der Herstellung;
- Figur 3: eine mikroskopische Aufnahme einer Ausführungsform der erfindungsgemäßen Zubereitung 6 h nach der Herstellung;
- Figur 4: eine mikroskopische Aufnahme einer Ausführungsform der erfindungsgemäßen Zubereitung 24 h nach der Herstellung und
- Figur 5: eine mikroskopische Aufnahme einer Ausführungsform der erfindungsgemäßen Zubereitung 4 Tage nach der Herstellung und anschließender Aufbewahrung im Kühlschrank bei 4 bis 8°C.

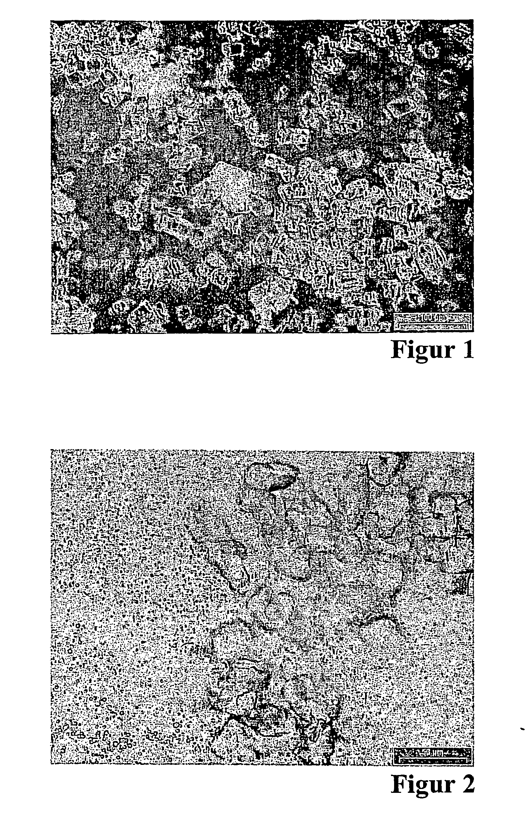

Die mikroskopische Aufnahme der Figur 1 zeigt eine Ingel-Mamyzin®-Suspension, die unmittelbar vor der Aufnahme hergestellt wurde. Hierzu wurden die handelsüblich gelieferten 2 Fläschchen, eine Flasche mit Methyl-4-hydroxybenzoat haltigem Wasser als Lösungsmittel und die andere Flasche mit der Trockensubstanz Penethamathydrojodid zusammengegeben und geschüttelt. Die erhaltene Suspension wurde mit einer Spritze entnommen und ein Flüssigkeitstropfen auf den Objektträger eines Mikroskops aufgebracht. Die Aufnahme zeigt ein intaktes System ohne erkennbare Zersetzung des suspendierten Penethamathydrojodids.

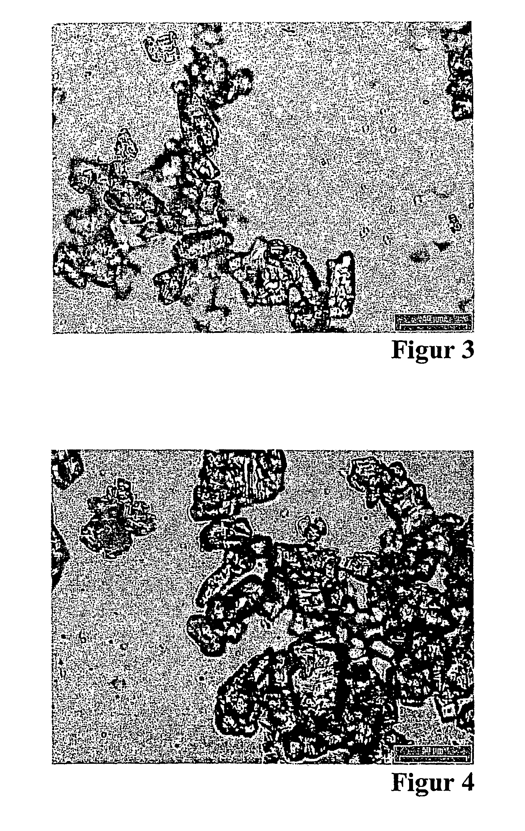

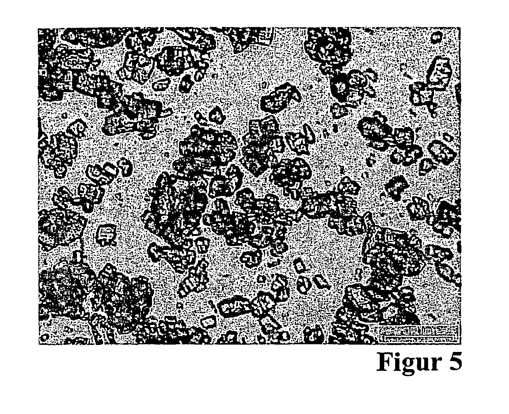

Die Figuren 2 bis 5 zeigen eine Ausführungsform der flüssigen Zubereitung der Erfindung in Abhängigkeit von der Aufbewahrungsdauer. Die Zubereitung wurde hergestellt aus 15,6 ml einer 2%igen Meloxicam-Lösung, zu der 5 g Penethamathydrojodid als Trockensubstanz zugegeben wurden. Anschließend wurde geschüttelt bis sich eine homogene Mischung in Form einer Suspension gebildet hatte. Die erhaltene Suspension wurde mit einer Spritze entnommen und ein Flüssigkeitstropfen auf den Objektträger eines Mikroskops aufgebracht. Die Proben wurden jeweils unmittelbar nach der Herstellung, nach 6 h, 24 h, und 4 Tage nach der Herstellung bei einer Aufbewahrung im Kühlschrank bei einer Temperatur von 4 bis 8°C entnommen. Sämtliche Aufnahmen zeigen ein intaktes System ohne erkennbare Zersetzung der Komponenten. Die erhaltene Suspension veränderte sich unabhängig von der Aufbewahrungszeit praktisch nicht; die Suspension lag daher auch noch nach 4 Tagen stabil vor und war damit uneingeschränkt verwendbar.

### Beispiel 2

Es sollte der Einfluß des alkalischen pH-Wertes und des Lösungsmittels auf die Stabilität des Penethamats überprüft werden. Dazu wurden eine erfindungsgemäße Suspension, hergestellt aus Meloxicam/Penethamathydrojodid in Ethanol/Wasser (Probelösung), und eine Vergleichslösung, die nach üblicher Herstellung aus den beiden Komponenten des Ingel-Mamyzins®: Lösungsmittel + Trockensubstanz erhalten wurde, mit einer speziell entwickelten HPLC-Methode untersucht und jeweils der Gehalt an Penethamat, Meloxicam und Impurity II bestimmt. Bei Impurity II handelt es sich um ein Zersetzungsprodukt von Meloxicam, d.h. um 2-Amino-5-methylthiazol, dessen steigender Gehalt eine zunehmende Zersetzung anzeigt.

### (a) HPLC-Methode

Folgende HPLC-Methode wurde verwendet:

| | |
|---|---|
| Säule: | YMC ODS AQ, 120 A, 3 µm, 150 x 4 mm, ohne Vorsäule |
| Fluß: | 0,8 ml/min |
| Temperatur: | 40°C |
| Detektion: | 260 nm |
| DAD: | Kontrolle Peakreinheit, Aufnahme von 190-600 nm |
| Injektionsvol.: | 10 µl |
| Mobile Phase: | Acetonitril/Puffer (33/67, V/V) |
| Puffer: | 2,7 g (0,02 mol) KH₂PO₄ und 4,4 g (0,02 mol) 1-Heptansulfonsäure- |
| | Natriumsalz-Monohydrat werden in ca. 800 ml bidest. Wasser (HPLC-Grad) gelöst. Die Lösung wird mit konz. Phosphorsäure auf pH = 3 eingestellt und dann auf 1000 ml aufgefüllt. |

### (b) Herstellung der Vergleichslösung

(Ingel-Mamyzin®: Lösungsmittel + Trockensubstanz)

Die Herstellung der Vergleichslösung erfolgte nach folgendem Verfahren:

### Herstellung der Stammlösung:

1 Flasche Ingel-Mamyzin®-Trockensubstanz (5g Penethamathydrojodid) wird mit 1 Flasche Ingel-Mamyzin®-Lösungsmittel (15,6 ml Wasser, konserviert mit 23,4 mg Methyl-4-hydroxybenzoat pro Flasche) versetzt und unter kräftigem Schütteln (ca. 2 min) so gut wie möglich gelöst. Diese Suspension wurde mit Acetonitril quantitativ in einen 100 ml Meßkolben überführt, mit 20 ml Placebolösung (2% Meloxicam) versetzt und mit Acetonitril auf 100,0 ml aufgefüllt.

Die Verdünnung wurde wie folgt durchgeführt:
1,0 ml der frisch bereiteten Stammlösung wurde mit Laufmittel (Acetonitril/Puffer 33/67, V/V) zu 50,0 ml verdünnt. Es ergaben sich folgende Konzentrationen:
c(Penethamat) = 1,0 mg/ml
c(Methyl-4-hydroxybenzoat) = 0,00468 mg/ml

### (c) Herstellung der Probelösung

- erfindungsgemäße flüssige Zubereitung -
(Ingel-Mamyzin®-Trockensubstanz + Meloxicam + Lösungsmittel)

Die Herstellung der Probelösung erfolgte nach folgendem Verfahren:

### Herstellung der Stammlösung:

1 Flasche Ingel-Mamyzin®-Trockensubstanz (5g Penethamathydrjodid) wurde mit 15,6 ml einer 2%igen Meloxicam-Lösung versetzt und unter kräftigem Schütteln (ca. 2 min) so gut wie möglich gelöst. Diese Suspension wurde mit Acetonitril quantitativ in einen 100 ml Meßkolben überfiihrt, mit 20 ml Placebolösung (2% Meloxicam) versetzt und mit Acetonitril auf 100,0 ml aufgefüllt.

Die Verdünnung wurde wie folgt durchgeführt:
1,0 ml der frisch bereiteten Stammlösung wurde mit Laufmittel (Acetonitril/Puffer 33/67, V/V) zu 50,0 ml verdünnt. Es ergaben sich folgende Konzentrationen:
c(Penethamat) = 1,0 mg/ml
c(Meloxicam) = 0,0624 mg/ml

### (d) Stabilitätsuntersuchung

Die Untersuchung der Gebrauchsstabilität, d.h. der Stabilität der Suspension unmittelbar nach der Herstellung, wurde als Doppelbestimmung an Probe- und Vergleichslösung bei 2 Lagerungsbedingungen (Kühlschrank und Raumtemperatur) nach einem vorgegeben Zeitfenster durchgeführt.

Die HPLC-Prüfungen für den Gehalt an Penethamat, Meloxicam und Impurity II erfolgten nach 0 (= Start), 2 und 7 Tagen gegen eine externe Kalibrierlösung.

Entsprechend dem Prüfdesign wurde jeweils 1 ml der Probe- und Vergleichslösung an Tag 1, 2 und 5 entnommen und verworfen. Bei der Auswertung wurde die entnommene Menge zur gefundenen Konzentration addiert. Das verwendete Prüfdesign ist in Tabelle 1 dargestellt:

**Tabelle 1: Verwendetes Prüfdesign**

| Parameter | Start | 1 Tag | 2 Tage | 5 Tage | 7 Tage |
|---|---|---|---|---|---|
| 2 bis 8°C | x | je 1 ml entnehmen und verwerfen | je 1 ml entnehmen und verwerfen | je 1 ml entnehmen und verwerfen | x |
| 5 Vergleichslsg. (TS¹⁾+Lösungsmittel) und 5 Probelösungen (TS+15,6 ml 2% Meloxicamlsg.) ansetzen | Je 2 Stamm- und Probelösungen herstellen und chromatographieren | 15,6 ml - 1 ml = 14,6 ml | 14,6 ml - 1 ml = 13,6 ml | 13,6 ml - 1 ml = 12,6 ml | Je 2 Stamm- und Probelösungen herstellen und chromatographieren (es verbleibt 1 Flasche als Reserve) |
| 15 bis 25°C | - | je 1 ml entnehmen und verwerfen | x | Versuch abgeschlossen | Versuch abgeschlossen |
| 3 Vergleichslsg. (TS+Lösungsmittel) und 3 Probelösungen (TS+15,6 ml 2% Metacamlsg.) ansetzen | - | 15,6 ml - 1 ml = 14,6 ml | Je 2 Stamm- und Probelösungen herstellen und chromatographieren (es verbleibt 1 Flasche als Reserve) | | |
| Bemerkung | Analyse der Vergleichs- und Probelösung | - | Analyse der Vergleichs- und Probelösung | - | Analyse der Vergleichs- und Probelösung |

| | | | | | |
|---|---|---|---|---|---|
| ¹⁾Trockensubstanz | | | | | |

### (e) Herstellung der Kalibrierlösung

Gegen folgende Kalibrierlösung wurde ausgewertet (n = 2):

### Herstellung der Stammlösung Impurity II:

Ca. 2,4 mg Impurity II wurden in 20,0 ml bidest. Wasser gelöst.
Die Konzentration betrug c = 0,12 mg/ml.

Es wurden ca. 100 mg Penethamat und ca. 6 mg Meloxicam in einen 10 ml Meßkolben eingewogen, mit jeweils 2 ml Acetonitril und Placebolösung (2% Meloxicam) und mit 0,5 ml Impurity II-Stammlösung versetzt (ggf. im Ultraschall-Bad behandelt) und mit Acetonitril zu 10,0 ml aufgefüllt.

1,0 ml dieser Lösung wird mit Laufmittel (Acetonitril/Puffer 33/67, VN) zu 10,0 ml verdünnt. Die erhaltenen Konzentrationen waren wir folgt:
c(Penethamat) = 1,0 mg/ml
c(Meloxicam) = 0,06 mg/ml
c(Impurity II) = 0,0006 mg/ml

### (f) Zusammenfassung der Ergebnisse

Die nachfolgende Tabelle 2 gibt eine Übersicht über die Ergebnisse (Mittelwerte einer Doppelbestimmung mit Doppelinjektion) der 3 Analyten zu den Untersuchungszeitpunkten t = 0, 2 und 7 Tagen.

**Tabelle 2: Zusammenfassung der Ergebnisse**

| | | Penethamat | | Meloxicam | | Impurity II | |
|---|---|---|---|---|---|---|---|
| | | Vgl.lsg. [%] | Probelsg. [%] | Vgl.lsg. [%] | Probelsg. [%] | Vgl.lsg. c[mg/ml] | Probelsg. c[mg/ml] |
| t = 0 | RT¹⁾ | 97,00 | 98,04 | - | 97,81 | - | 0,0001175 |
| t = 2 | RT¹⁾ | 96,58 | 91,64 | - | 98,69 | - | 0,0001318 |
| t = 7 | KS²⁾ | 99,78 | 99,47 | - | 101,92 | - | 0,0001129 |

| | | | | | | | |
|---|---|---|---|---|---|---|---|
| ¹⁾Raumtemperatur ²⁾Kühlschrank | | | | | | | |

Die Untersuchungen der bei 2-8°C gelagerten Proben ergaben nach einer Lagerungsdauer von 7 Tagen keine Abnahme des Gehaltes an Penethamat und Meloxicam.

Bei Lagerung bei Raumtemperatur nahm der Gehalt an Penethamat um ca. 6,4 % im Vergleich zur Startuntersuchung (t = 0) ab. Die beiden anderen Analyten Meloxicam und Impurity II erwiesen sich als stabil.

Zur Kontrolle der Peakreinheit wurden DAD-Spektren im Wellenlängenbereich von 190 bis 600 nm aufgenommen und der Match-Faktor ermittelt. Bei einem Match-Faktor > 900 ist die Peakreinheit gegeben. Sämtliche Match-Faktoren lagen über 900.

## Patentansprüche

1. Flüssige Zubereitung für die Veterinärmedizin, enthaltend
- als ersten Wirkstoff Meloxicam,
- als zweiten Wirkstoff Penethamathydrojodid und
- mindestens ein Lösungsmittel.

2. Flüssige Zubereitung nach Anspruch 1, **dadurch gekennzeichnet, dass** Meloxicam im Lösungsmittel gelöst vorliegt.

3. Flüssige Zubereitung nach Anspruch 1 oder 2, **dadurch gekennzeichnet, dass** das Lösungsmittel ausgewählt ist aus der Gruppe, bestehend aus Alkohol, Wasser und einer Alkohol/Wasser Mischung.

4. Flüssige Zubereitung nach einem der Ansprüche 1, 2 oder 3, **dadurch gekennzeichnet, dass** der Alkohol Ethanol darstellt.

5. Flüssige Zubereitung nach einem der Ansprüche 1 bis 4, **dadurch gekennzeichnet, dass** das Lösungsmittel ein Ethanol/Wasser-Gemisch mit einem Verhältnis Ethanol zu Wasser von 1:7 bis 1:5,7 darstellt.

6. Flüssige Zubereitung nach einem der Ansprüche 1 bis 5, **dadurch gekennzeichnet, dass** die Zubereitung 0,2 bis 0,5 g Penethamathydroiodid pro ml Endformulierung aufweist.

7. Flüssige Zubereitung nach einem der vorangehenden Ansprüche 1 bis 6, **dadurch gekennzeichnet, dass** Meloxicam in der flüssigen Zubereitung in einer Menge von 0,2 bis 0,4 g pro ml vorhanden ist.

8. Flüssige Zubereitung nach einem der vorangehenden Ansprüche 1 bis 7, **dadurch gekennzeichnet, dass** in der Zubereitung ein oder mehrere übliche Hilfsstoffe vorliegen.

9. Flüssige Zubereitung nach einem der vorangehenden Ansprüche 1 bis 8, **dadurch gekennzeichnet, dass** die Zubereitung eine intramuskuläre Injektionslösung darstellt.

10. Verfahren zur Herstellung der Zubereitung nach einem der Ansprüche 1 bis 9, umfassend die Schritte:
(a) Lösen von Meloxicam in einem Lösungsmittel;
(b) Zugeben von Penethamathydrojodid zu der in Schritt (a) erhaltenen Lösung und
(c) Schütteln der in Schritt (b) erhaltenen Mischung unter Erhalt der flüssigen Zubereitung in Form einer Suspension.

11. Verfahren nach Anspruch 10, **dadurch gekennzeichnet, dass** in Schritt (a) bereits eine handelsübliche Meloxicam-Injektionslösung eingesetzt wird.

12. Verfahren nach einem der Ansprüche 11 bis 11, **dadurch gekennzeichnet, dass** Penethamathydrojodid als Trockensubstanz eingesetzt wird.

13. Verfahren nach einem der Ansprüche 11 bis 12, **dadurch gekennzeichnet, dass** die flüssige Zubereitung unmittelbar vor Gebrauch hergestellt wird.

14. Verwendung einer flüssigen Zubereitung nach einem der Ansprüche 1 bis 9 zur Herstellung eines Arzneimittels zur Behandlung oder Vorbeugung von Krankheiten bei Tieren, ausgewählt aus den Indikationen. Atemwegserkrankungen bei Rindern und Kühen, Durchfallerkrankungen bei Kälbern, Mastitis, akute Mastitis der Milchkuh, Mastitis-Metritis-Agalactica-Syndrom des Schweins, nicht infektiösen Lahmheiten beim Schwein, Prostatitis und dem chronischen Schmerzsyndrom im kleinen Becken.

15. Verwendung nach Anspruch 14, **dadurch gekennzeichnet dass** die Krankheit Mastitis darstellt.

## Claims

1. Liquid preparation for veterinary medicine, containing
- as first active substance meloxicam,
- as second active substance penethamate hydroiodide and
- at least one solvent.

2. Liquid preparation according to claim 1, **characterised in that** meloxicam is present dissolved in the solvent.

3. Liquid preparation according to claim 1 or 2, **characterised in that** the solvent is selected from among alcohol, water and an alcohol/water mixture.

4. Liquid preparation according to one of claims 1, 2 or 3, **characterised in that** the alcohol is ethanol.

5. Liquid preparation according to one of claims 1 to 4, **characterised in that** the solvent is a mixture of ethanol and water with a ratio of ethanol to water of 1:7 to 1:5.7.

6. Liquid preparation according to one of claims 1 to 5, **characterised in that** the preparation contains 0.2 to 0.5 g penethamate hydroiodide per ml of final formulation.

7. Liquid preparation according to one of the preceding claims 1 to 6, **characterised in that** meloxicam is present in the liquid preparation in an amount of from 0.2 to 0.4 g per ml.

8. Liquid preparation according to one of the preceding claims 1 to 7, **characterised in that** there are one or more conventional excipients in the preparation.

9. Liquid preparation according to one of the preceding claims 1 to 8, **characterised in that** the preparation is a solution for intramuscular injection.

10. Process for producing the preparation according to one of claims 1 to 9, comprising the steps of:
(a) dissolving meloxicam in a solvent;
(b) adding penethamate hydroiodide to the solution obtained in step (a) and
(c) shaking the mixture obtained in step (b) to obtain the liquid preparation in the form of a suspension.

11. Process according to claim 10, **characterised in that** a standard commercial injectable meloxicam solution is used in step (a).

12. Process according to one of claims 11 to 11, **characterised in that** penethamate hydroiodide is used as a dry substance.

13. Process according to one of claims 11 to 12, **characterised in that** the liquid preparation is produced immediately before use.

14. Use of a liquid preparation according to one of claims 1 to 9 for preparing a pharmaceutical composition for the treatment or prevention of diseases in animals, selected from among the indications of respiratory diseases in cattle and cows, diarrhoea in calves, mastitis, acute mastitis in dairy cows, mastitis-metritis-agalactica syndrome in pigs, non-infectious lameness in pigs, prostatitis and chronic pain syndrome in the small pelvis.

15. Use according to claim 14, **characterised in that** the disease is mastitis.

## Revendications

1. Préparation liquide pour la médecine vétérinaire contenant
- comme premier principe actif du méloxicam,
- comme second principe actif de l'iodhydrate de pénéthamate et
- au moins un solvant.

2. Préparation liquide selon la revendication 1 **caractérisée en ce que** le méloxicam est présent dissous dans le solvant.

3. Préparation liquide selon la revendication 1 ou 2 **caractérisée en ce que** le solvant est choisi dans le groupe consistant en un alcool, l'eau et un mélange alcool/eau.

4. Préparation liquide selon l'une des revendications 1, 2 ou 3 **caractérisée en ce que** l'alcool représente l'éthanol.

5. Préparation liquide selon l'une des revendications 1 à 4 **caractérisée en ce que** le solvant est un mélange éthanol/eau avec un rapport de l'éthanol à l'eau de 1 : 7 à 1 : 5,7.

6. Préparation liquide selon l'une des revendications 1 à 5 **caractérisée en ce que** la préparation comporte 0,2 à 0,5 g d'iodhydrate de pénéthamate par ml de formulation finale.

7. Préparation liquide selon l'une des revendications 1 à 6 précédentes **caractérisée en ce que** le méloxicam dans la préparation liquide est présent en une quantité de 0,2 à 0,4 g par ml.

8. Préparation liquide selon l'une des revendications 1 à 7 précédentes **caractérisée en ce qu'**un ou plusieurs adjuvants courants sont présents dans la préparation.

9. Préparation liquide selon l'une des revendications 1 à 8 précédentes **caractérisée en ce que** la préparation représente une solution pour injection intramusculaire.

10. Procédé pour produire la préparation selon l'une des revendications 1 à 9 comprenant les étapes :
(a) dissolution du méloxicam dans un solvant ;
(b) addition d'iodhydrate de pénéthamate à la solution obtenue dans l'étape (a) et
(c) agitation du mélange obtenu dans l'étape (b) avec obtention de la préparation liquide sous forme d'une suspension.

11. Procédé selon la revendication 10 **caractérisé en ce que**, dans l'étape (a), une solution pour injection de méloxicam du commerce est déjà utilisée.

12. Procédé selon l'une des revendications 11 à 11 **caractérisé en ce que** l'iodhydrate de pénéthamate est utilisé sous forme de substance sèche.

13. Procédé selon l'une des revendications 11 à 12 **caractérisé en ce que** la préparation liquide est produite juste avant l'emploi.

14. Utilisation d'une préparation liquide selon l'une des revendications 1 à 9 pour la production d'un médicament pour le traitement ou la prévention de maladies chez les animaux choisies parmi les indications maladies des voies respiratoires chez les boeufs et les vaches, maladies de type diarrhée chez les veaux, mastite, mastite aiguë de la vache laitière, syndrome de mastite-métrite-agalactie du porc, paralysies non infectieuses chez le porc, prostatite et le syndrome de douleur chronique dans le petit bassin.

15. Utilisation selon la revendication 14 **caractérisée en ce que** la maladie représente la mastite.
